# EUROPEAN PATENT APPLICATION

(11) **EP 3 838 310 A1**
(43) Date of publication of application: **23.06.2021**
(21) Application number: 19850302.1
(22) Date of filing: 13.08.2019
(51) Int. Cl.: A61M 5/142, A61M 5/158, A61M 25/06

(54) **NEEDLE ASSEMBLY AND DRUG INJECTION DEVICE INCLUDING SAME**

(30) Priority: 14.08.2018 KR 20180095161
(71) Applicant: Eoflow Co., Ltd., Bundang-gu, Seongnam-si Gyeonggi-do 13605 (KR)
(72) Inventor: KIM, Jesse Jae Jin, Seongnam-si Gyeonggi-do 13628 (KR); KIM, Seung Ha, Goyang-si Gyeonggi-do 10243 (KR); PARK, Dae Jong, Seoul 03943 (KR)
(74) Representative: Gulde & Partner
(86) International application number: PCT/KR2019/010270
(87) International publication number: WO 2020/036402

(57) **Abstract**

The present disclosure provides a needle assembly and a drug injection device including a needle, a cannula into which the needle is inserted, a first holder supporting the needle, a second holder disposed to face one side of the first holder and supporting the cannula, a sleeve accommodating the first holder in an inner space and rotating with respect to the first holder, and an elastic member disposed between the first holder and the sleeve, wherein, when the sleeve is rotated, the elastic member is expanded and the first holder and the second holder are moved to an extended position, and wherein, when the sleeve is further rotated, the elastic member is contracted and only the first holder is returned to an original position.

## Description

### [TECHNICAL FIELD]

The present disclosure relates to a device, and more particularly, to a needle assembly and a drug injection device including the same.

### [BACKGROUND ART]

In general, drug injection devices such as insulin injection devices are used to inject drugs into the patient's body. The drug injection device is also used by professional medical staff such as doctors and nurses, but in most cases, it is used by the general public such as the patient or guardian. In diabetic patients, especially pediatric diabetic patients, drugs such as insulin need to be injected into the human body at predetermined intervals. Therefore, drug injection devices in the form of patches that are attached to the human body for a certain period of time are being developed. The drug injection device may be used in a state attached to the human body such as the abdomen or waist of the patient in the form of the patch for the predetermined period.

The drug injection device needs to have excellent fit when attached to the human body, needs to be convenient to use, needs to be excellent in durability, and needs to be driven with low power. In particular, since the drug injection device is attached to the patient's skin and used, a user should insert a needle or cannula into the patient's skin simply and conveniently.

### [DESCRIPTION OF EMBODIMENTS]

### [TECHNICAL PROBLEM]

The present disclosure provides a needle assembly and a drug injection device, which allows a user to operate simply and accurately.

### [SOLUTION TO PROBLEM]

The present disclosure provides a needle assembly including a needle, a cannula into which the needle is inserted, a first holder supporting the needle, a second holder disposed to face one side of the first holder and supporting the cannula, a sleeve accommodating the first holder in an inner space and rotating with respect to the first holder, and an elastic member disposed between the first holder and the sleeve, wherein, when the sleeve is rotated, the elastic member is expanded and the first holder and the second holder are moved to an extended position, and wherein, when the sleeve is further rotated, the elastic member is contracted and only the first holder is returned to an original position.

### [ADVANTAGEOUS EFFECTS OF DISCLOSURE]

According to an embodiment of the present disclosure, a user may easily and simply use the needle assembly and the drug injection device. The user may insert the needle and the cannula by rotating the sleeve, fix a position of the cannula and easily withdrawn only the needle from the cannula by further rotating the sleeve.

In addition, in the case of the needle assembly, since a flexible locking protrusion is fixed to a ledge part of a housing, which is an external structure, the cannula may be fixed at an exact position after being inserted into a skin. Since the ledge part supports the second holder, the cannula may be stably supported.

In addition, the needle assembly includes a sensor having conductivity, and whether the cannula is correctly inserted may be confirmed by contacting the sensor. Of course, the scope of the present disclosure is not limited by these effects.

### [DESCRIPTION OF DRAWINGS]

FIG. 1 is a diagram showing a drug injection device according to an embodiment of the present disclosure.
FIG. 2 is a plan view showing the internal components of the drug injection device of FIG. 1.
FIG. 3 is an exploded perspective view showing a needle assembly of FIG. 2.
FIG. 4 is a perspective view showing a second holder of FIG. 3.
FIGS. 5A to 5c are cross-sectional views showing the operation of the needle assembly.
FIGS. 6A and 6B are diagrams showing a coupling relationship between a second holder and a cannula illustrated in FIG. 3.
FIG. 7 is a diagram showing a first holder according to another embodiment of the present disclosure.

### [BEST MODE]

The present disclosure provides a needle assembly including a needle, a cannula into which the needle is inserted, a first holder supporting the needle, a second holder disposed to face one side of the first holder and supporting the cannula, a sleeve accommodating the first holder in an inner space and rotating with respect to the first holder, and an elastic member disposed between the first holder and the sleeve, wherein, when the sleeve is rotated, the elastic member is expanded and the first holder and the second holder are moved to an extended position, and wherein, when the sleeve is further rotated, the elastic member is contracted and only the first holder is returned to an original position.

Also, the second holder may include a locking protrusion extending outward and being flexible, and when the elastic member is expanded, the locking protrusion may be supported by an external structure installed outside the needle assembly.

Also, a side surface of the locking protrusion may have an inclined surface inclined with respect to an axial direction.

Also, the second holder may have a sensor disposed on one surface of the locking protrusion and sensing contact of the locking protrusion.

Also, the first holder may have a guide groove disposed on an outer surface of the first holder, and an inner surface of the sleeve may have a movable protrusion that moves along the guide groove.

Also, the sleeve may be rotated in a first direction to move the cannula and the needle to the extended position, and thereafter, the sleeve may be rotated in the first direction or in a second direction opposite to the first direction to take out the needle from the cannula.

The present disclosure provides a drug injection device including a base, a housing disposed above the base, a sleeve rotatable about a first axis with respect to the housing, a first holder inserted into an inner space of the sleeve, supporting a needle, and moving along the first axis, a second holder disposed on one side of the first holder and supporting the cannula, and an elastic member disposed between the first holder and the sleeve, wherein, when the sleeve is rotated, the first holder and the second holder are moved in one direction along the first axis, and the second holder is fixed to the housing, and wherein, when the sleeve is further rotated, only the first holder is moved in the opposite direction along the first axis.

Also, the second holder may have a flexible locking protrusion extending outward, and the housing may have a ledge part protruding from one side of the housing to support the locking protrusion.

Also, a side surface of the locking protrusion may have an inclined surface inclined with respect to an axial direction.

Also, the second holder may have a sensor disposed on one surface of the locking protrusion and sensing contact with the base.

Other aspects, features, and advantages other than those described above will become apparent from the following drawings, claims, and detailed description of the invention.

### [MODE OF DISCLOSURE]

In the present disclosure, various transformations may be applied and various embodiments may be provided, and specific embodiments will be illustrated in the drawings and described in detail in the detailed description. Effects and features of the present disclosure, and a method of achieving them will be apparent with reference to embodiments described later in detail together with the drawings. However, the present disclosure is not limited to the embodiments disclosed below and may be implemented in various forms.

Hereinafter, embodiments of the present disclosure will be described in detail with reference to the accompanying drawings, and when describing with reference to the drawings, the same or corresponding constituent elements are assigned the same reference numerals, and redundant descriptions thereof will be omitted.

In the following examples, expressions in the singular include plural expressions unless the context clearly indicates otherwise.

In the following embodiments, terms such as include or have means that the features or elements described in the specification are present, and do not preclude the possibility of adding one or more other features or elements.

When a certain embodiment can be implemented differently, a specific process order may be performed differently from the described order. For example, two processes described in succession may be performed substantially simultaneously, or may be performed in an order opposite to the described order.

In the drawings, components may be exaggerated or reduced in size for convenience of description. For example, the size and thickness of each component shown in the drawings are arbitrarily shown for convenience of description, and thus the following embodiments are not necessarily limited to those shown.

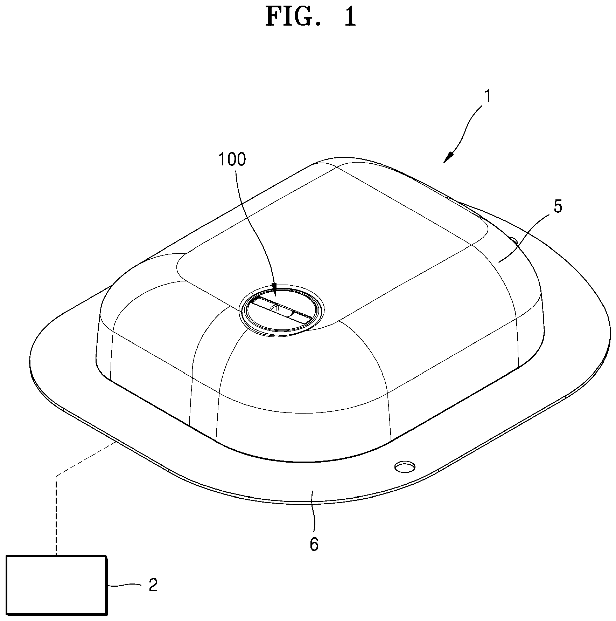

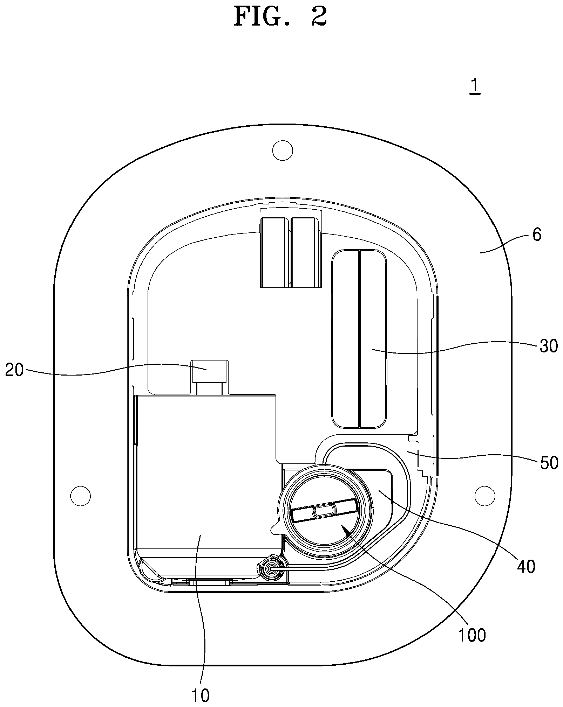

FIG. 1 is a diagram showing a drug injection device 1 according to an embodiment of the present disclosure, and FIG. 2 is a plan view showing the internal components of the drug injection device 1 of FIG. 1.

Referring to FIGS. 1 and 2, a drug injection device 1 may be connected to a remote device 2 connected by wire or wirelessly. A user may use the drug injection device 1 by manipulating the remote device 2, and may monitor the states of use of the drug injection device 1. For example, the user may monitor the amount of the drug injected from the drug injection device 1, the number of injects of the drug, the amount of the drug stored in a storage tank 10, the user's bio information, etc., and may drive the drug injection device 1 based on this.

The drug injection device 1 may include an outer cover 5 surrounding an upper outer portion and an attachment portion 6 attached to the user's skin, and a plurality of parts are mounted in a space between the outer cover 5 and the attachment portion 6.

In an inner space of the drug injection device 1, the storage tank 10 in which the drug is stored, a drive shaft 20 driven to inject the drug into the storage tank 10 or discharge the drug from the storage tank 10, and a drive unit 30 for transmitting power to the drive shaft 20 may be disposed. In addition, the drug injection device 1 may include a base 40 disposed above the attachment portion 6 and a housing 50 disposed above the base 40, and a needle assembly 100 may be supported by the housing 50.

Inside the storage tank 10, a plunger (not illustrated) is connected to the drive shaft 20, and when the drive shaft 20 moves in an axial direction, the drug may be discharged to the needle assembly 100 by the plunger.

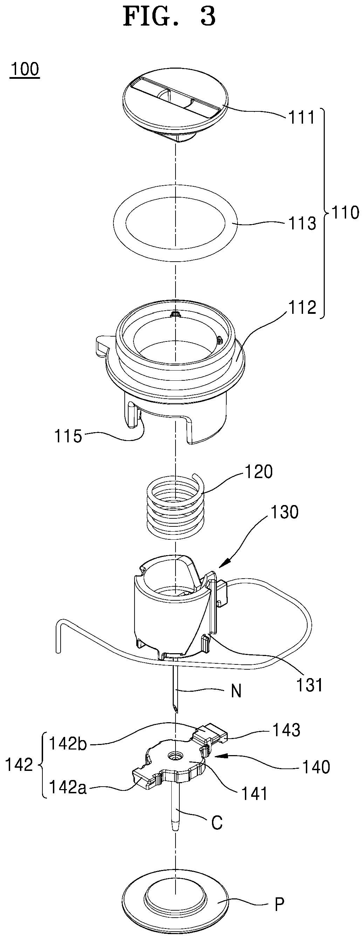

FIG. 3 is an exploded perspective view showing a needle assembly 100 of FIG. 2.

Referring to FIG. 3, the needle assembly 100 may include a sleeve 110, an elastic member 120, a first holder 130, a second holder 140, a needle N, a cannula C and a patch P.

The sleeve 110 forms an outer appearance of the needle assembly 100 and may be rotated about a longitudinal direction as a central axis. The sleeve 110 may include a knob 111, a rotating body 112, and a supporter ring 113.

The knob 111 is mounted on an upper end of the rotating body 112 and may have a groove on an upper surface. The user may be able to rotate the sleeve 110 by manipulating the groove of the knob 111 exposed to an outside. The elastic member 120 is disposed on a lower surface of the knob 111, and the knob may receive an expansion force when the elastic member 120 expands in an axial direction.

The rotating body 112 has a substantially cylindrical shape, and an opening of the upper end may be covered by the knob 111. The supporter ring 113 may be mounted on an outer upper end of the rotating body 112.

The elastic member 120, the first holder 130, and the second holder 140 may be disposed in an inner space of the rotating body 112. A movable protrusion 115 may be disposed on an inner surface of the rotating body 112. The movable protrusion 115 may be moved along a guide groove 131 disposed on an outer peripheral surface of the first holder 130. When the rotating body 112 is rotated, the movable protrusion 115 may move along the sidewalls of the guide groove 131 so that the first holder 130 may be raised or lowered.

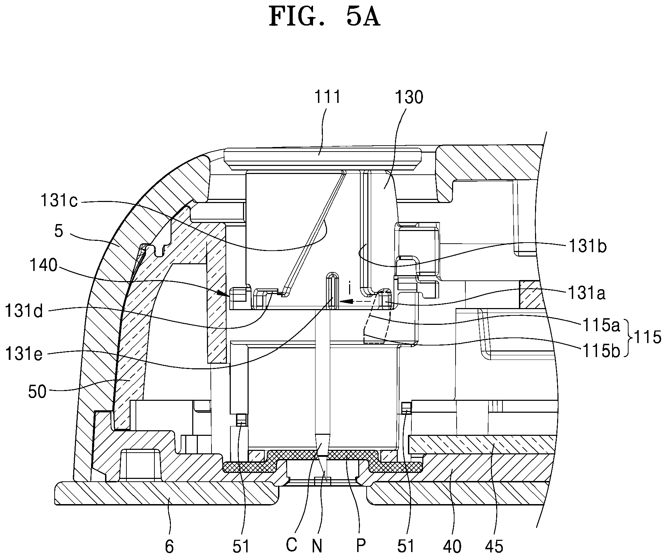

Referring to FIG. 5A, the movable protrusion 115 may include an inclined wall 115A and a vertical wall 115b. The inclined wall 115A may induce a stable contact when descending along the guide groove 131, and the vertical wall 115b may contact a movement limiting groove 131e to define a moving path of the movable protrusion 115.

The elastic member 120 may be disposed between the sleeve 110 and the first holder 130. When the elastic member 120 is expanded, the first holder 130 may be moved downward. The elastic member 120 is not limited to a specific member, and may be made of various members that transmit elastic force in the longitudinal direction. However, in the following description, for convenience of description, the elastic member 120 will be mainly described in the case of a spring type.

The first holder 130 may support the needle N. Since the needle N is inserted and fixed to one side of the first holder 130, when the first holder 130 is moved in the axial direction, the needle N is also moved. The first holder 130 is disposed in an inner space of the sleeve 110, and the elastic member 120 is disposed above it.

Referring to FIG. 5A, the first holder 130 may include the guide groove 131 on the outer surface. The guide groove 131 may guide the movement of the movable protrusion 115. When the sleeve 110 is rotated, the movable protrusion 115 is moved to a preset path formed by the guide groove 131 so that the first holder 130 may be raised or lowered.

The guide groove 131 may include a first stopping groove 131a, a lifting groove 131b, an inclined groove 131c, a second stopping groove 131d, and the movement limiting groove 131e. The first stopping groove 131a is a groove in which the movable protrusion 115 is initially positioned, and when the movable protrusion is positioned in the first stopping groove, the elastic member 120 is maintained in a contracted state. The lifting groove 131b extends in the longitudinal direction of the first holder 130. When the elastic member 120 expands, the first holder 130 is lowered, and the movable protrusion 115 is moved along the lifting groove 131b. The inclined groove 131c extends to have a predetermined slope. When the movable protrusion 115 is moved along the inclined groove 131c, the elastic member 120 contracts again, and the first holder 130 is raised again. The second stopping groove 131d is a groove in which the movable protrusion 115 returns to its initial position, and when the movable protrusion is positioned in the second stopping groove, the elastic member 120 is maintained in a contracted state again.

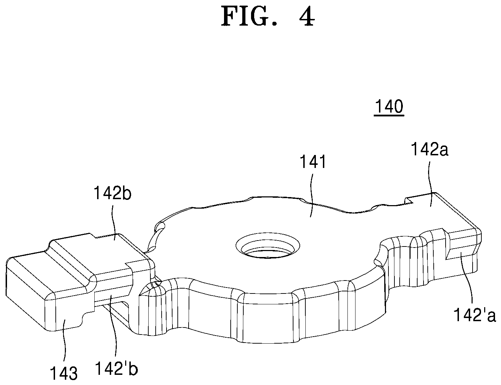

FIG. 4 is a perspective view showing a second holder 140 of FIG. 3.

Referring to FIGS. 3 and 4, the second holder 140 is disposed to face one side of the first holder 130 and may support the cannula C. The second holder 140 may include a central body 141, a locking protrusion 142, and a sensor 143.

The second holder 140 is formed of a flexible material, and may be instantly deformed when an external force is applied. However, when the external force applied to the second holder is removed, the original shape is restored again. The second holder 140 is in contact with the first holder 130 at an initial position, but when the elastic member 120 is expanded, it is separated from the first holder 130 and fixed to the housing 50.

The cannula C may be inserted into a center of the central body 141, and the needle N may be selectively coupled. The central body 141 may contact a lower end of the first holder 130.

The locking protrusion 142 may protrude outward from the central body 141 and may be formed to be flexible. The locking protrusion 142 may be provided in plural. For example, the locking protrusion 142 may include a first protrusion 142a and a second protrusion 142b disposed to face each other.

The locking protrusion 142 may be fixed to the housing 50 according to the movement of the second holder 140. When the elastic member 120 expands, the locking protrusion 142 may be fixed to an external structure disposed outside the needle assembly 100, that is, the housing 50.

The locking protrusion 142 may have an inclined surface inclined with respect to the axial direction on a side surface. The first protrusion 142a has a first inclined surface 142'a, and the second protrusion 142b has a second inclined surface 142'b. Since the locking protrusion 142 has a trapezoidal shape due to the inclined surface, when the locking protrusion 142 moves downward, it may be fixed to the ledge part 51 by easily crossing the ledge part 51 of the housing 50. A detailed description of this will be described later.

The sensor 143 may be disposed on one side of the locking protrusion 142. However, the present disclosure is not limited thereto, and the sensor 143 may be disposed on an outer surface of the central body 141. The sensor 143 may detect whether the cannula C is accurately inserted into the skin of the user. The sensor 143 may sense a contact between the second holder 140 and the base 40. In detail, the sensor may sense whether the second holder 140 moves downward through the ledge part 51 and contacts a contact portion 45 disposed above the base 40. Since the sensor 143 is formed of a conductive material, an electrical signal is generated when it comes into contact with the contact portion 45, and a control unit (not illustrated) may check whether the second holder 140 is in contact with the base 40.

Since the needle N is fixed to the first holder 130, it may be inserted or released into the cannula C by moving the first holder 130 in the axial direction. One end of the needle N may be connected to the storage tank 10 to receive the drug, and the other end may be inserted into the cannula C and moved along the cannula C.

Since the cannula C is fixed to the second holder 140, it may be inserted into the skin of the user by moving the second holder 140 in the axial direction. Since the cannula C has a conduit shape capable of accommodating the needle N, the drug discharged from the needle N may be injected into the user.

The patch P is supported on the base 40, and may fixe the position of the cannula C. Since an end of the cannula C is supported by the patch P, separation of the cannula C during storage or during transport may be prevented.

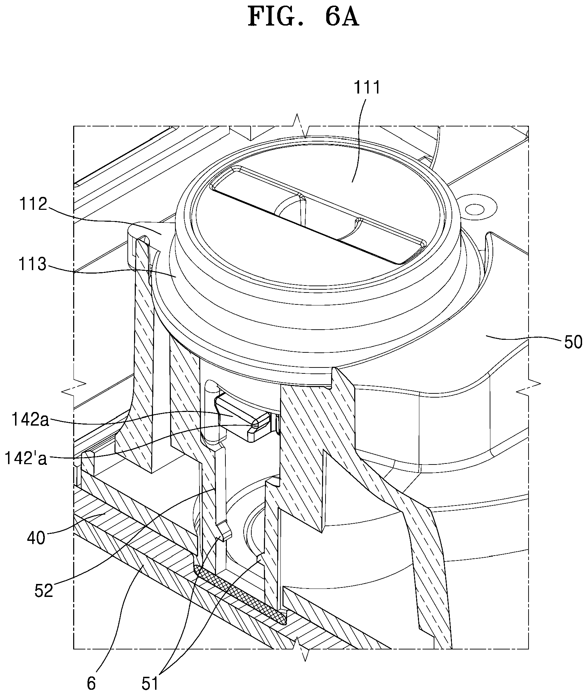

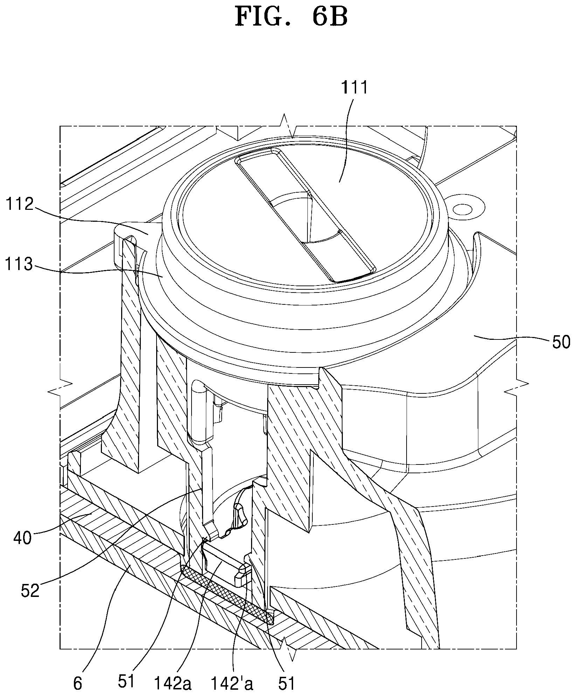

FIGS. 5A to 5C are cross-sectional views showing the operation of the needle assembly 100, and FIGS. 6a and 6b are diagrams showing a coupling relationship between a second holder 140 and a cannula C of FIG. 3.

Referring to FIGS. 5A to 6a, driving of the needle assembly 100 is performed as follows. In the needle assembly 100, when the sleeve 110 is rotated, the elastic member 120 is expanded, and the first holder 130 and the second holder 140 are moved to an extended position, and, when the sleeve 110 is further rotated in the same direction, the elastic member 120 contracts, and only the first holder returns to its original position. Thus, the needle N and the cannula C are inserted together into the skin of the user, and the needle N is then extracted from the cannula C. For convenience of understanding the operation, FIGS. 5A to 5C are illustrated with the rotating body 112 removed.

First, FIGS. 5A and 6A show the needle assembly 100 in its original position, that is, a position before the user uses the needle assembly 100. The movable protrusion 115 of the sleeve 110 is supported by the first stopping groove 131a, and the elastic member 120 is maintained in a compressed state between the knob 111 and the first holder 130. Accordingly, the first holder 130 and the second holder 140 are disposed above the needle assembly 100, and the needle N and the cannula C are maintained inside the drug injection device 1.

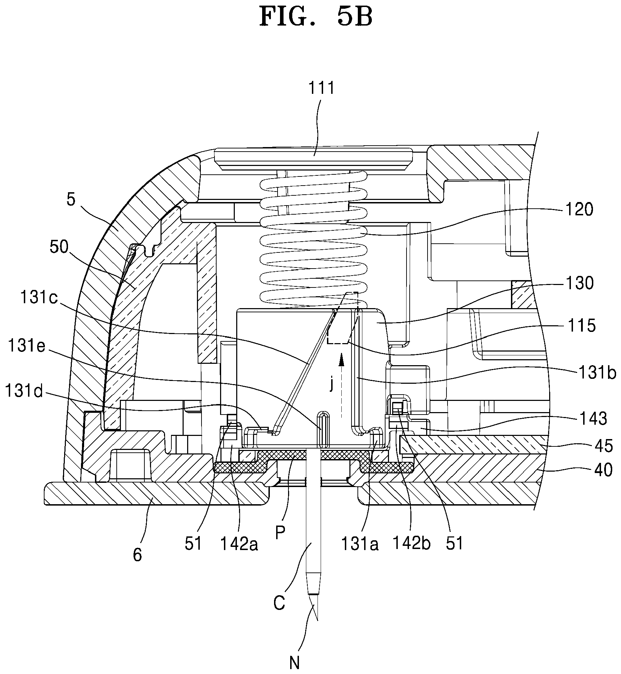

When the user rotates the knob 111 in one direction (i direction), the sleeve 110 is rotated in the one direction with respect to the first holder 130, and the movable protrusion 115 is moved beyond the first stopping groove 131a. Then, the elastic member 120 is expanded in the axial direction, and the vertical wall 115b of the movable protrusion 115 is moved along the lifting groove 131b (in a j direction) as shown in FIG. 5B, and at the same time, the first holder 130 and the second holder 140 moves downward along the guide opening 52 of the housing 50. Meanwhile, the movement limiting groove 131e may limit excessive movement of the movable protrusion 115 in a radial direction and may induce a vertical movement of the movable protrusion 115.

When the first holder 130 and the second holder 140 are moved downward, the needle N and the cannula C are inserted into the skin of the user. Since the first holder 130 applies a force to the second holder 140, the second holder 140 may pass through the ledge part 51 of the housing 50. Since the second holder 140 is formed of the flexible material, when the first holder 130 applies the force to the second holder 140, the locking protrusion 142 is instantly deformed and passes through the ledge part 51. In particular, since the first inclined surface 142'a of the first protrusion 142a and the second inclined surface 142'b of the second protrusion 142b may be slidably moved along an inclined surface of the ledge part 51, the locking protrusion 142 smoothly pass through the ledge part 51. Thereafter, a flat lower surface of the ledge part 51 is maintained in contact with a flat upper surface of the locking protrusion 142.

When the locking protrusion 142 passes through the ledge part 51, the sensor 143 is in contact with the contact portion 45. Since the sensor 143 is formed of the conductive material, the electric signal is generated when it comes into contact with the contact portion 45. When the sensor 143 contacts the contact portion 45, the control unit (not illustrated) receives the electric signal, and it can be recognized that the cannula C is normally inserted into the skin of the user.

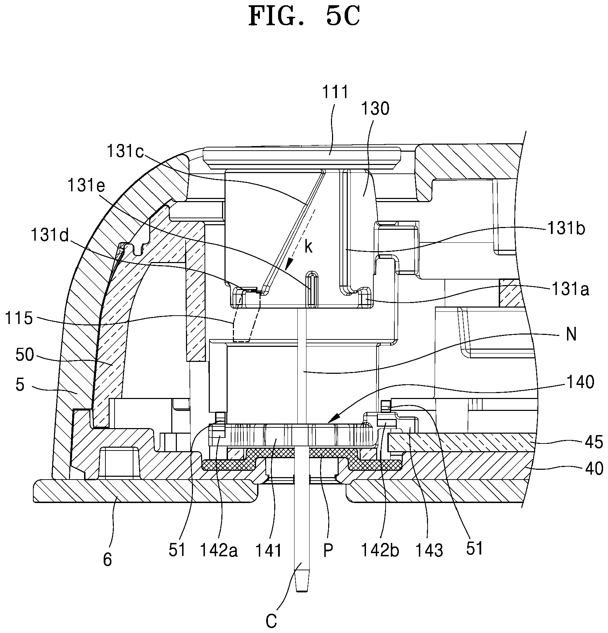

Thereafter, as illustrated in FIGS. 5c and 6b, when the user further rotates the knob 111 in the one direction, the sleeve 110 is rotated in the one direction with respect to the first holder 130, and the movable protrusion 115 is moved along guide groove 131 (in a k direction). When the inclined wall 115A of the movable protrusion 115 is moved along the inclined surface of the inclined groove 131c, the first holder 130 is raised again, and the elastic member 120 is compressed again.

The flat upper surface of the locking protrusion 142 is in contact with the flat lower surface of the ledge part 51, and the ledge part 51 limits the elevation of the second holder 140. The second holder 140 is separated from the first holder 130 and is not lifted, however, the first holder 130 is raised again by the movable protrusion 115, and the needle N is withdrawn from the cannula C.

Thereafter, the drug is discharged from the storage tank 10 to the needle N, so that the drug may be injected into the user through the cannula C.

The needle assembly 100 and the drug injection device 1 having the same according to the present disclosure may be easily and simply used by the user. The user may insert the needle and the cannula by rotating the sleeve in the one direction, and by further rotating the sleeve in the one direction, may fix the position of the cannula and easily withdraw only the needle from the cannula.

In addition, in the needle assembly 100, since the flexible locking protrusion 142 is fixed to the ledge part 51 of the housing 50, which is the external structure, the cannula C may be fixed at an exact position after it is inserted into the skin. Since the ledge part 51 supports the second holder 140, the cannula C may be stably supported.

In addition, the needle assembly 100 includes the sensor 143 having conductibility, and it is possible to check whether the cannula C is correctly inserted by contacting the sensor 143.

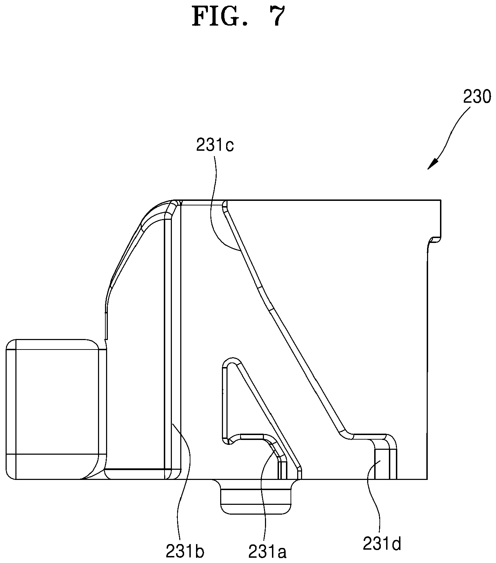

FIG. 7 is a diagram showing a first holder according to another embodiment of the present disclosure.

Referring to FIG. 7, a guide groove of a first holder 230 has a shape different from that of the guide groove 131 of the above-described embodiment.

The guide groove 231 may include a first stopping groove 231a, a lifting groove 231b, an inclined groove 231c, and a second stopping groove 231d. The first stopping groove 231a is a groove in which the movable protrusion 115 is initially located, and when the movable protrusion is positioned in the first stopping groove, the elastic member 120 is maintained in a contracted state. The lifting groove 231b extends in a longitudinal direction of the first holder 230. When the elastic member 120 is expanded, the first holder 230 is lowered, and the movable protrusion 115 is moved along the lifting groove 231b. The inclined groove 231c is extended to have a predetermined slope. When the movable protrusion 115 is moved along the inclined groove 231c, the elastic member 120 contracts again, and the first holder 230 is raised again. The second stopping groove 231d is a groove in which the movable protrusion 115 returns to its initial position, and when the movable protrusion is positioned in the second stopping groove, the elastic member 120 is maintained in a contracted state again.

When the sleeve is rotated in one direction, the movable protrusion 115 crosses the first stopping groove 231a and contacts the lifting groove 231b. The lifting groove 231b limits a rotation distance of the movable protrusion 115, and the movable protrusion 115 may be moved in a vertical direction along the lifting groove 231b. Since the first holder 230 and the second holder move together in a downward direction, the needle and the cannula are inserted into the skin of the user.

Then, when the sleeve is moved in the other direction, the movable protrusion 115 is moved along the inclined groove 231c. At this time, since the elastic member is compressed again and only the first holder 230 is moved to the sleeve, the needle may be withdrawn from the cannula. Thereafter, the movable protrusion 115 is inserted and fixed to the second stopping groove 231 d.

The first holder 230 may separate the insertion of the needle and the cannula and the separation of the needle by changing the rotation direction. In addition, the first holder 230 may limit the rotation distance of the movable protrusion 115 by the lifting groove 231 b, so that it may prevent the user from excessively rotating the sleeve to incompletely insert the cannula.

Accordingly, the spirit of the present disclosure is limited to the above-described embodiments and should not be defined, and not only the claims to be described later, but also all ranges equivalent to or equivalently changed from the claims are the spirit of the present disclosure. I would say it belongs to the category.

### [INDUSTRIAL APPLICABILITY]

The needle assembly and the drug injection device including the same according to an embodiment of the present disclosure may be applied to various industrially available devices. The needle assembly and the drug injection device including the same according to an embodiment of the present disclosure may be applied to a device for delivering various drugs.

## Claims

1. A needle assembly comprising:
a needle;
a cannula into which the needle is inserted;
a first holder supporting the needle;
a second holder disposed to face one side of the first holder and supporting the cannula;
a sleeve accommodating the first holder in an inner space and rotating with respect to the first holder; and
an elastic member disposed between the first holder and the sleeve, wherein, when the sleeve is rotated, the elastic member is expanded and the first holder and the second holder are moved to an extended position, and wherein, when the sleeve is further rotated, the elastic member is contracted and only the first holder is returned to an original position.

2. The needle assembly of claim 1, wherein the second holder includes a locking protrusion extending outward and being flexible, and wherein, when the elastic member is expanded, the locking protrusion is supported by an external structure installed outside the needle assembly.

3. The needle assembly of claim 2, wherein a side surface of the locking protrusion has an inclined surface inclined with respect to an axial direction.

4. The needle assembly of claim 2, wherein the second holder has a sensor disposed on one surface of the locking protrusion and sensing contact of the locking protrusion.

5. The needle assembly of claim 1, wherein the first holder has a guide groove disposed on an outer surface of the first holder, and
wherein an inner surface of the sleeve has a movable protrusion that moves along the guide groove.

6. The needle assembly of claim 1, wherein the sleeve is rotated in a first direction to move the cannula and the needle to the extended position, and thereafter, the sleeve is rotated in the first direction or in a second direction opposite to the first direction to take out the needle from the cannula.

7. A drug injection device comprising:
a base;
a housing disposed above the base;
a sleeve rotatable about a first axis with respect to the housing;
a first holder inserted into an inner space of the sleeve, supporting a needle, and moving along the first axis;
a second holder disposed on one side of the first holder and supporting the cannula; and
an elastic member disposed between the first holder and the sleeve, wherein, when the sleeve is rotated, the first holder and the second holder are moved in one direction along the first axis, and the second holder is fixed to the housing, and
wherein, when the sleeve is further rotated, only the first holder is moved in the opposite direction along the first axis.

8. The drug injection device of claim 7, wherein the second holder has a flexible locking protrusion extending outward, and
wherein the housing has a ledge part protruding from one side of the housing to support the locking protrusion.

9. The drug injection device of claim 8, wherein a side surface of the locking protrusion has an inclined surface inclined with respect to an axial direction.

10. The drug injection device of claim 8, wherein the second holder has a sensor disposed on one surface of the locking protrusion and sensing contact with the base.
